# EUROPEAN PATENT SPECIFICATION

(11) **EP 1 343 882 B1**
(45) Date of publication and mention of the grant of the patent: **23.06.2004**
(21) Application number: 01271437.4
(22) Date of filing: 13.12.2001
(51) Int. Cl.: C12N 15/10

(54) **COMPOSITIONS, METHODS AND KITS FOR IDENTIFYING PROTEIN-PROTEIN INTERACTION DISRUPTING AGENTS**
Zusammensetzungen, Verfahren und Reagentiensätze zur Erkennung von Protein-Protein Wechselwirkung störenden Substanzen
COMPOSITIONS, PROCEDES ET KITS PERMETTANT D'IDENTIFIER DES AGENTS PERTURBATEURS DES INTERACTIONS PROTEINE-PROTEINE

(30) Priority: 18.12.2000 US 255910 P
(43) Date of publication of application: 17.09.2003
(73) Proprietor: National Research Council of Canada, Ottawa, Ontario K1A 0R6 (CA)
(72) Inventor: ZHAO, Hui-Fen, Lasalle, Quebec H8N 3A6 (CA); SHEN, Shi-Hsiang, Beaconsfield, Quebec H9W 1C2 (CA)
(74) Representative: Prins, Adrianus Willem, Mr. Ir.
(86) International application number: PCT/CA2001/001770
(87) International publication number: WO 2002/050259

(56) References cited:
- US-A- 5 925 523
- GOSSEN M ET AL: "TIGHT CONTROL OF GENE EXPRESSION IN MAMMALIAN CELLS BY TETRACYCLINE-RESPONSIVE PROMOTERS" PROCEEDINGS OF THE NATIONAL ACADEMY OF SCIENCES OF USA, NATIONAL ACADEMY OF SCIENCE. WASHINGTON, US, vol. 89, no. 12, 15 June 1992 (1992-06-15), pages 5547-5551, XP000564458 ISSN: 0027-8424
- HITTELMANN A.B. ET AL.,: "differential regulation of glucorticoid receptor transcriptional activation via AF-1-associated proteins" EMBO JOURNAL, vol. 18, no. 19, 1999, pages 5380-5388, XP002202270
- CHAPPELL S.A. ET AL.,: "A 9nt segment of a cellular mRNA can function as an internal ribosome entry site (ires) and when present in linked multiple copies greatly enhances ires activity" PROC. NATL. ACAD. SCI, USA, vol. 97, no. 4, 15 February 2000 (2000-02-15), pages 1536-1541, XP002202271 cited in the application
- SHIODA T. ET AL.,: "a green fluorescnet protein-reporter mammalian two-hybrid system with extrachromosomal maintenace of a prey expression plasmid: application to interaction screening" PROC. NATL. ACAD. SCI., USA, vol. 97, no. 10, 9 May 2000 (2000-05-09), pages 5220-5224, XP002202272 cited in the application
- FINLEY R L ET AL: "TWO-HYBRID ANALYSIS OF GENETIC REGULATORY NETWORKS. THE YEAST TWO-HYBRID SYSTEM" YEAST TWO-HYBRID SYSTEM ( ADVANCES IN MOLECULAR BIOLOGY ), XX, XX, 1997, pages 197-214, XP002922642

## Description

### FIELD OF THE INVENTION

The present invention relates to compositions, methods, and kits for identifying agents that are capable of disrupting protein-protein interaction in mammalian cells.

### BACKGROUND OF THE INVENTION

Protein-protein interactions are involved in most biological processes and cellular functions such as the formation of cellular macromolecular structures, receptor and enzyme complexes, and in the regulation of signal transduction pathways. Elucidation of protein-protein interactions in the cell also constitutes an important approach to functional genomics. The most powerful technology for the detection of protein-protein interactions is the yeast two-hybrid system, which was first described by Fields and Song (Nature 340: 245-246. 1989).

Protein-protein interactions can be disrupted by certain molecules such as peptides or drugs. At present, the selection of these disrupting molecules for a particular protein-protein interaction is executed using yeast reverse two-hybrid systems (Leanna & Hannink. 1996. Nucleic Acids Res. 24, 3341-347; Vital et al. 1996. Proc. Natl. Acad. Sci. USA. 93, 10315-10320; Shih, et al. 1996. Proc. Natl. Acad. Sci. USA.93, 13896-13901) or a bacterial reverse two-hybrid system (Zhang et al. 2000. Nature Biotech. 18,71-74; Park & Raines. 2000. Nature Biotech. 18, 847-851). Although the yeast and bacterial reverse two-hybrid systems are useful for some genetic selections, they are not ideal systems for screening therapeutic molecules such as drugs as the permeability of these therapeutic molecules in yeast or in bacteria is very different from that in mammalian cells because of difference in cellular compartmentation among yeast, bacteria and mammalian cells. In fact, the potential of drug candidates needs to be evaluated in mammalian cells where such drugs will be used eventually. In addition, various post-translational modifications required for particular protein-protein interactions are limited in both yeast and bacteria. In a mammalian system post-translational modification will occur in a natural fashion for particular protein-protein interactions. Presently, there are a few reports of mammalian two-hybrid systems for protein-protein interactions (Luo et al. 2000. US Patent 6,114,111 Shioda et al. 2000. Proc. Natl. Acad. Sci. USA 97, 5220-5224).

However, none of these mammalian systems are suitable for high throughput reverse two-hybrid screening of disrupting agents such as therapeutic molecules because in these mammalian systems expression of the two interactive proteins was not regulated. To perform high throughput screening of potential therapeutic molecules that disrupt protein-protein interactions in mammalian cells, it is desirable that therapeutic molecules be introduced into the mammalian system before the synthesis of the two interactive proteins is induced in either stably or transiently transfected cells. In the prior systems, constitutive expression of the two interactive proteins can immediately activate the reporter gene. Consequently, the inhibitory effect, for example, of a drug on blocking the protein interaction cannot be evaluated accurately by the repressive extent of the reporter gene as the reporter gene has already been fully expressed before addition of the drug in the system. Theoretically, the constructs for expressing the two interactive proteins may be transiently transfected into the cells with the drugs in each well for screening. However, performing such transient transfection of the cells with the construct in each drug-containing well is practically impossible for high throughput drug screening because one person may only be able to transfect constructs into a few 96-well plates per day.

### SUMMARY OF THE INVENTION

Accordingly, a mammalian reverse two-hybrid system for screening protein-protein interaction dissociating agents was developed. The system is particularly useful for high throughput screening of small molecules that prevent protein-protein interactions. The two interactive proteins are fused either with a DNA binding domain or with a transcriptional activation domain of a transcription factor. The expression of the two interactive fusion proteins is regulated by a suppressor which is expressed constitutively in the cells. In the presence of an inducer, the repression of the promoter directing the expression of the two interactive fusion proteins is lifted, leading to the expression of two interactive fusion proteins. Once the two interactive fusion proteins are produced, the DNA binding domain of one fusion protein binds to a DNA binding element in a promoter and brings the transcription activation domain of the other interactive fusion protein to the promoter via protein-protein interaction and thereby activates the transcription of the reporter gene. In the presence of an inducer and a protein-protein interaction dissociating agent, the two interactive proteins fail to interact. Thus, the transcription of the reporter gene is not activated. As a result, the non-expression of the reporter gene is taken as an indicator of identification of an agent such as a small therapeutic drug molecule capable of dissociating protein-protein interaction.

Both the reporter protein coding construct and the suppressor construct are typically integrated into chromosomes of the cells. The construct coding for the two interactive fusion proteins is also preferably stably transfected into cells. For high throughput analysis, the constructs of interactive fusion proteins can also be transiently transfected into a batch of multi-million cells that are already stably transfected with the reporter protein coding construct and the suppressor coding construct. These cells can then be divided into multiple wells of plates containing test agents and an inducer for identifying protein-protein interaction dissociating agent and/or protein-protein interaction non-dissociating agent in a high throughput manner. Since the suppressor construct via constitutive expression of a hybrid-repressor protein tightly regulates the expression of the interactive fusion proteins; the transient transfection of cells with the interactive fusion protein constructs does not result in the expression of the interactive fusion proteins and subsequent activation of the reporter gene until an inducer is added. Further, the reporter gene will only be expressed in situations where a test agent cannot cause disruption of protein-protein interaction.

The present invention provides a mammalian reverse two-hybrid system for identifying a change in protein-protein interaction comprising: a suppressor construct comprising a constitutive promoter operably linked to a repressor protein coding sequence; an interactive fusion protein coding construct comprising a promoter operably linked to a repressible operator repressible by a repressor protein coded by said repressor protein coding sequence, a first interactive protein coding sequence fused in frame with a transcriptional activation domain protein coding sequence and a second interactive protein coding sequence fused in frame with a DNA binding domain protein coding sequence; and, a reporter construct comprising a DNA binding element bindable to said DNA binding domain protein operably linked to a minimal promoter and a reporter coding sequence.

In a preferred embodiment the interactive fusion protein coding construct can itself be made up of or derived from a first interactive protein-transcriptional activation domain fusion protein coding construct comprising a promoter operably linked to a repressible operator repressible by a repressor protein coded by said repressor protein coding sequence and a first interactive protein coding sequence fused in frame with a transcriptional activation domain protein coding sequence; and, a second interactive protein-DNA binding domain fusion protein coding (for consistency, we use "coding" rather than "gene") construct comprising a promoter operably linked to a repressible operator repressible by a repressor protein coded by said repressor protein coding sequence, a second interactive protein coding sequence fused in frame with a DNA binding domain protein coding sequence.

Preferably the mammalian reverse two-hybrid system additionally comprises at least one internal ribosome entry site (IRES) sequence. In a particularly preferred embodiment said first interactive protein coding sequence fused in frame with a transcriptional activation domain protein coding sequence is operably linked to said second interactive protein coding sequence fused in frame with a DNA binding domain protein coding sequence through an internal ribosome entry site (IRES) sequence. The IRES may typically be a synthetic IRES sequence or an encephalomyocarditis virus IRES.

The reporter construct may additionally comprise a second reporter coding sequence operably linked (preferably through an IRES) to the already-present (first) reporter sequence. In a preferred embodiment in said reporter construct said promoter is selected from the group consisting of a minimal promoter from human cytomegalovirus (CMV) promoter, SV40 early promoter, adenovirus E1b promoter, respiratory syncytial virus (RSV) promoter, EF1 promoter and thymine kinase (TK) promoter.

The present invention also comprises plasmids comprising a mammalian reverse two-hybrid system of the invention. In a preferred embodiment there is provided a set of two plasmids wherein said suppressor construct and said reporter construct are provided on a first plasmid and said first interactive protein-transcriptional activation domain fusion protein coding construct and said second interactive protein-DNA binding domain fusion protein coding construct together are provided on a second plasmid.

The invention further comprises a mammalian cell comprising a mammalian reverse two-hybrid system of the invention, preferably integrated into a chromosome of the cell.

The invention further comprises a method for identifying a change in a protein-protein interaction which comprises subjecting a mammalian reverse two-hybrid system of the invention in a cell to an inducer and a test protein-protein interaction disrupting agent; and assaying said cell for expression or non-expression of said reporter coding sequence, non-expression of said reporter coding sequence being indicative of disrupting ability of said test protein-protein interaction disrupting agent.

In this method the disrupting agent may be an indirect disrupting agent being tested for an ability to inhibit activity of a disease (e.g. cancer) - related enzyme such as a kinase.

Alternatively the method described above may comprise (a) stably introducing a suppressor construct and a reporter construct in a cell; and (b)(i) stably introducing a first interactive protein-transcriptional activation domain fusion protein coding construct and a second interactive protein-DNA binding domain fusion protein coding construct into said cell; or (b)(ii) transiently introducing a first interactive protein-transcriptional activation domain fusion protein coding construct and a second interactive protein-DNA binding domain fusion protein coding construct into said cell; (c) subjecting a cell resulting from step (b)(i) or (b) (ii) to an inducer and a test protein-protein interaction disrupting agent; (d) assaying said subjected cell for the non-expression and expression of said reporter coding sequence; and, (e) identifying said protein-protein interaction disrupting agent based on non-expression of said reporter coding sequence.

In another aspect the invention provides a kit for identifying a change in protein-protein interaction comprising: one or more aliquots of a suppressor construct, a first interactive protein-transcriptional activation domain fusion protein coding construct, a second interactive protein-DNA binding domain fusion protein coding construct, and a reporter construct on one or more plasmids; and, one or more aliquots of a reagent, cell, and apparatus needed for conducting a method for identifying a change in protein-protein interaction.

The kit may comprise one or more aliquots of one or more plasmids as defined, one or more aliquots of a cell containing a construct as defined and appropriate reagents and apparatus.

### BRIEF DESCRIPTION OF THE DRAWINGS

**Figure 1A-1E** illustrates an outline of various constructs used and principle involved in executing the present invention.
**Figure 2** shows disruption of protein-protein interaction between FKBP12 and transforming growth factor β (TGFβ) receptor RI in 293A cells by the drug molecule FK506.
**Figure 3** shows disruption of protein-protein interaction between epidermal growth factor receptor (EGFR) and p85 in 293A cells by the drug molecule AG1478.
**Figure 4** shows visual indication of disruption of protein-protein interaction between FKBP12 and the TGFβ receptor RI (Fig. 4a-4d) and between EGFR and p85 (Fig. 4e-4h) in 293A cells by the drug molecule FK506 and AG1478 by non-expression of the GFP reporter gene.
**Figure 5** shows use of β-lactamase as a reporter gene in the present invention.

### DETAILED DESCRIPTION OF THE INVENTION

The present invention provides compositions, methods, and kits pertaining to a mammalian reverse two-hybrid system particularly suitable for identifying agents that are capable of disrupting protein-protein interactions in mammalian cells. In a first embodiment, the identification of protein-protein interaction disrupting agents can be done in a high throughput manner. In another embodiment, the disrupting agents can be small molecules, peptides or any synthetic/natural occurring molecules with therapeutic effect. The protein-protein interaction disrupting agents may be those molecules that either directly or indirectly disrupt protein-protein interaction. In the case of indirect disruption, those molecules can inhibit the activity of a particular protein, for example, an enzyme. These enzymes may be kinases, preferably disease-related kinases. The word disruption also implies change.

The mammalian reverse two-hybrid system of the present invention typically has four components: a suppressor coding construct, a first interactive protein- transcriptional activation domain fusion protein coding construct, a second interactive protein-DNA binding domain fusion protein coding construct, and a reporter protein coding construct. These can be assembled into hybrid constructs comprising two or more of the above components.

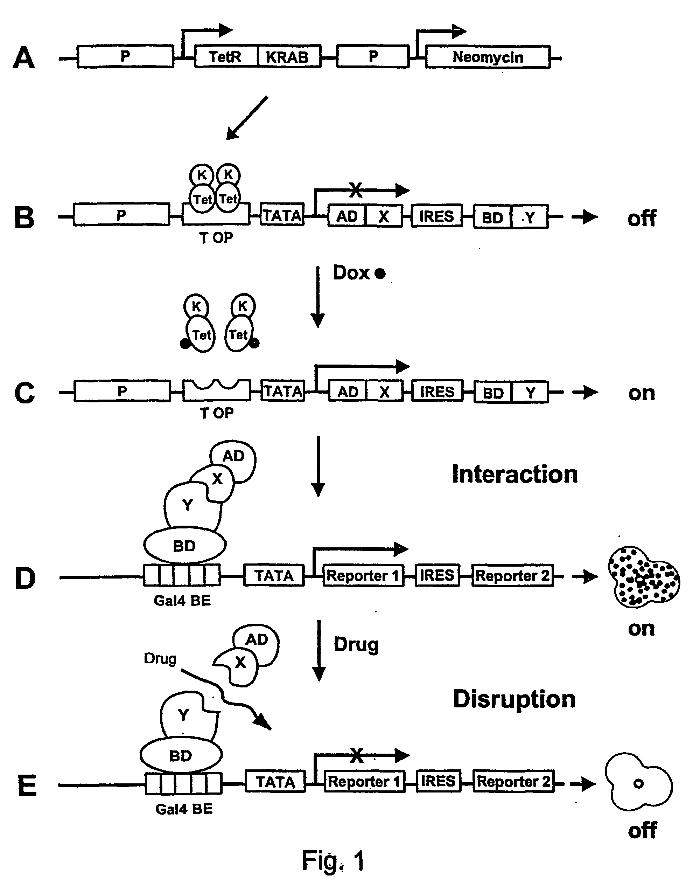

The suppressor construct typically comprises, in 5' to 3' direction and operably linked, a constitutive promoter for regulating transcription of a coding sequence downstream of the promoter, a sequence coding for a hybrid-repressor protein, and a constitutive promoter regulating a sequence coding for a selective marker such as a drug selective marker e.g., neomycin, hygromycin, puromycin, or any other drug selective markers useable in mammalian cells (see Fig 1). Cell lines used in the present invention can be from a mammal, such as a mouse, a rat, a primate, or a human. Suitable cells for executing protein-protein interactions of present invention may, for example, be selected from CHO, Cos, NIH 3T3, Hela, and human embryonic kidney 293 cells.

A constitutive promoter is a cis-regulatory element capable of regulating the expression of a coding sequence downstream of it in a continuous manner. Such constitutive promoters are know in the prior art and may be selected, for example, from human cytomegalovirus (CMV), SV40, respiratory syncytial virus (RSV), EF1 and thymine kinase (TK) promoters. In an embodiment described below, the constitutive promoter is a promoter from the immediate-early gene promoter of human cytomegalovirus (CMV).

A hybrid-repressor protein, in the absence of an inducer, is capable of binding to an operator in a promoter and thereby repressing the expression of a coding sequence downstream of the promoter. Such repressor proteins are know in the prior art and may be selected from Lac, λ, and Tet repressors from *Escherichia coli.* The tetracycline repressor (TetR) protein can bind to its operator inserted in a promoter located upstream of a coding sequence and can block the transcription of the coding sequence. In mammalian cells, however, the TetR protein has been found not to completely block the transcriptional activity of the promoter. Thus, in an embodiment described below, a hybrid-TetR protein capable of completely blocking the transcription of a coding sequence downstream of a promoter (Deuschle et al. 1995. Mol. Cell Biol. 15, 1907-1914) is used. The repressor TetR protein used in the present invention is a hybrid-repressor in which the TetR coding sequence is linked to a nuclear localization sequence (NLS) which in turn is linked to the KRAB domain of the human transcriptional suppressor Kox1gene (Deuschle et al. 1995. Mol. Cell Biol. 15, 1907-1914). The repression can be lifted by providing an inducer, which binds to the repressor TetR protein, thereby freeing the promoter for transcribing the coding sequence downstream of the promoter. Such inducers are know in the prior art and may be selected from lactose for the lac repressor and tetracycline for the tetracycline repressor. In an embodiment described below, doxycycline (Dox), a derivative of tetracycline, is used as the inducer. Doxycycline binds to the hybrid-TetR protein, thereby preventing the binding of the hybrid-TetR protein to the operator sequence, resulting in the transcription of the coding sequence downstream of the promoter. In an embodiment described below, the coding sequence is either a first interactive protein fused to a transcriptional activation domain or a second interactive protein fused to a DNA binding domain. In a preferred embodiment, the coding sequence can be a first interactive protein fused to an activation domain and a second interactive protein fused to a DNA binding domain linked by an internal ribosome entry site (IRES) sequence (Chappell et al., 2000, Proc. Natl. Acad. Sci. USA 97,1536-1541). This synthetic IRES is more active than the commonly used encephalomyocarditis virus (EMCV) IRES, and thereby it ensures appropriate expression levels of the two interactive proteins regulated by a common promoter upstream of the interactive protein coding sequences.

The first interactive protein-transcriptional activation domain fusion protein coding construct typically comprises, in 5' to 3' direction and operably linked, an inducible promoter with an operator, a coding sequence for a first interactive protein fused in frame to a coding sequence of a transcriptional activation domain (see Fig 1) and an IRES sequence linking to a sequence coding for a second interactive protein fused with a DNA binding domain. The transcriptional activation domain is capable of recruiting the transcriptional machinery for the transcription of a reporter gene upon interaction of second interactive protein with the first interactive protein. Such interaction between the first interactive protein and the second interactive protein is disruptable by certain protein-protein interaction disrupting agents, leading to the non-expression of the reporter gene.

An inducible promoter, in the absence of an inducer, is normally repressed by a repressor protein thereby unabling the promoter to transcribe a coding sequence downstream of it. However, in the presence of an inducer, the inducible promoter is able to express a coding sequence downstream of it. Such inducible promoters are know in the prior art and may be selected from the Lac, the lambda P(L), Tet and the tryptophan operon from *Escherichia coli* in conjunction with a mammalian core promoter. In an embodiment described below, the inducible promoter is a core CMV promoter (most, if not all of the promoter except the TATA box) linked to a tetracycline operator (T OP). This promoter is normally repressed in the presence of constitutively expressed TetR-NLS-KRAB hybrid-repressor protein but is able to express a coding sequence downstream of it in the presence of the inducer doxycycline (DOX).

A first interactive protein is the first of the two interactive proteins that is fused in frame to an activation domain of a transcription factor. Such first interactive proteins may be selected, for example, from any partners of interacting proteins that are important for cellular signal transductions and cellular processes such as receptors, adaptors, affectors and enzymes, and may be a disease-related signalling molecule. By adaptor we mean a protein without catalytic or transcriptional activity, but as a scaffold it has domains binding to other proteins. By affector we mean a protein mediating a signal transduction pathway. Further such first interactive proteins may also be identified in future and fused with the activation domain of a transcription factor. In embodiments described below, the first interactive protein is either a transforming growth factor type I receptor (TGFβR) (RI) or the p85 subunit of phosphatidylinositol 3-kinase. The activation domains of a transcription factor are also known in the prior art and may be selected from GAL4, B42, VP16, and NF-kB p65. In embodiments described below, the activation domain of a transcription factor is a VP16 that interacts, with the help of first and second interactive proteins, with a minimal promoter to activate transcription of a reporter gene.

A second interactive protein-DNA binding domain fusion protein coding construct typically comprises, in 5' to 3' direction and operably linked, an inducible promoter with an operator as described above, a coding sequence for a second interactive protein fused in frame to the coding sequence of a DNA binding domain. The DNA binding domain is capable of binding to a nucleic acid element upstream of a minimal promoter provided in a reporter protein coding construct as described below.

The second interactive protein is the interactive protein that is fused in frame to a DNA binding domain. Such a second interactive protein may be selected from proteins that interact with the first interactive proteins.

They include, but are not limited to, proteases, phosphatases, kinases, receptors, and other enzyme proteins. In particular, kinases may be disease-related kinases such as EGFR, PDGFR Her2, VEGFR, Met, FGFR, IGFR insulin receptor, JAKs, PKA, PKC, MAP kinase, PDGFR, Her2, VEGFR, Met, FGFR, IGFR, insulin receptor, JAKs, PKA, PKC, MAP kinase, ZAP70, v-Abl, as well as Lck, Lyn, and other Src-like kinases. Further, such second interactive proteins may also be identified in future and fused with the DNA binding domain. In embodiments described below, the second interactive protein is either the immunophilin FKPB 12 or an epidermal growth factor receptor (EGFR).

The DNA binding domains are also known in the prior art and may be selected from Tet repressor, lambda repressor, Lax A DNA binding sequence, NF-kB p65, and p53. In embodiments described below, the DNA binding domain is a Gal4 binding sequence from *S. cerevisiae* that binds to a Gal4 binding element inserted in the upstream of the minimal promoter, such as cytomegaloviral (CMV) promoter.

In a preferred embodiment described below, the first interactive protein-transcriptional activation domain fusion protein coding construct and the second interactive protein- DNA binding domain fusion protein coding construct are combined on the same plasmid. In such a case, the coding sequence for the first interactive protein is fused in frame to the coding sequence of the transcriptional activation domain and the coding sequence for the second interactive protein is fused to the coding sequence of the DNA binding domain and then the two components are linked by a synthetic internal ribosome entry site (IRES) sequence (Chappell et al. 2000 Proc. Natl. Acad. Sci. USA 97,1536-1541). In this way, expression of the two interacting proteins can be regulated by the same promoter upstream of the two interactive protein coding sequences.

A reporter construct typically comprises, in 5' to 3' direction and operably linked, a DNA binding element, a minimal promoter, and a reporter coding sequence.

The DNA binding element is bound by the DNA binding domain of the second interactive protein-DNA binding domain fusion protein. In embodiments described below, the DNA binding element is a Gal4 DNA binding element.

A reporter gene can be used to indirectly detect the occurrence of a reaction. In the present invention the non-expression of the reporter gene is used as an indication of disruption between two interactive proteins. Such reporter genes are know in the prior art and may be selected, for example, from genes for green fluorescent protein (GFP) and derivatives, luciferase, β-lactamase, β-galactosidase, alkaline phosphatase, chloramphenicol acetyl transferase, and drug selective markers such as neomycin, hygromycin, puromycin, or any other drug selective markers useable in mammalian cells. In embodiments described below, the following reporter genes were used: a green fluorescent protein (GFP) gene, a β-lactamase gene, and a β-galactosidase gene. In a preferred embodiment, two or more reporter genes are linked, together by the IRES sequence, for easy visual evaluation (for GFP) and enhanced biochemical evaluation (for β-lactamase gene or β-galactosidase gene).

The four components of the mammalian reverse two-hybrid system of the present invention described above may be present on one or more plasmids. In one embodiment, the suppressor coding construct and the reporter protein coding constructs are provided on a first plasmid and the two interactive protein fusion constructs are provided on a second plasmid.

Fig. 1 illustrates the basic principles of the invention through an embodiment of the present invention.

Fig. 1A shows an example of a suppressor construct used for enhanced suppression of the promoter (P) in interactive protein constructs. The following elements were operably linked in 5' to 3' direction: a CMV promoter (P), TetR coding sequence, NLS (not shown), KRAB domain of human Kox1 gene, and a selective marker coding sequence directed by a promoter (P).

Fig. 1B shows an example of an interactive fusion protein construct wherein both interactive proteins are linked via the internal ribosome entry site (IRES) sequence and are controlled by a common promoter. The following elements were operably linked in 5' to 3' direction: a core CMV promoter (P), tetracycline operator (T OP), TATA box from the CMV promoter, transcriptional activation domain (AD) fused to a first interactive protein (X), IRES, and DNA binding domain (BD) fused to a second interactive protein (Y). In the absence of an inducer, the tetracycline operator can be seen occupied by two units of TetR-KRAB repressor thus keeping the CMV promoter suppressed, resulting in non-expression of interactive fusion protein, depicted by "off."

Fig. 1C shows an example of an interactive fusion protein construct wherein both interactive proteins are linked via the IRES sequence and are controlled by a common promoter. The following elements were operably linked in 5' to 3' direction: a core CMV promoter (P), tetracycline operator (T OP), TATA box from the CMV promoter, transcriptional activation domain (AD) fused to a first interactive protein (X), IRES, and DNA binding domain (BD) fused to a second interactive protein (Y). In the presence of an inducer (Dox), the inducer interacts with the TetR-KRAB to free the tetracycline operator from two units of TetR-KRAB repressor thus lifting suppression of the CMV promoter, resulting in the transcription of the interactive fusion protein, depicted by "on."

Fig. 1D shows an example of a reporter construct wherein two reporter coding sequences are linked via an IRES sequence. The following elements were operably linked in 5' to 3' direction: four Gal 4 binding elements (Gal4 BE), a TATA box of the CMV minimal promoter; a first reporter coding sequence (Reporter 1), the IRES sequence, and a second reporter coding sequence (Reporter 2). In the presence of an inducer (DOX), but in the absence of a protein-protein interaction dissociating agent (drug), the DNA binding domain (BD) of the second interactive fusion protein binds to the Gal4 binding element and the first interactive fusion protein interacts with the second interactive fusion protein that brings the transcriptional activation domain (AD) of the first interactive-transcriptional activation domain fusion protein in contact with the DNA binding domain to activate the transcription of the reporter coding sequences, depicted by "on" and shown by the presence of dots in a cell.

Fig. 1E shows an example of a reporter construct wherein two reporter coding sequences are linked via an IRES sequence. The following elements were operably, linked in 5' to 3' direction: four Gal 4 binding elements (Gal4 BE), a TATA box of the CMV minimal promoter, a first reporter coding sequence (Reporter 1), the IRES sequence, and a second reporter coding sequence (Reporter 2). In the presence of an inducer (Dox) and a protein-protein interaction dissociating agent (shown as Drug), the interaction between the two interactive proteins is disrupted by the drug. In the absence of such a protein interaction, the transcription of the reporter coding sequence is not activated, depicted by "off' and shown by the absence of dots in a cell.

The components of the present invention as described above can be used in a method to identify protein-protein interaction disrupting agents in mammalian cells. This can be achieved by transfecting cells with the constructs described above: a suppressor construct, a first interactive protein-transcriptional activation domain fusion protein coding construct, a second interactive protein-DNA binding domain fusion protein coding construct, and a reporter construct; selecting cells for the integration or the presence of said constructs; subjecting the selected cells to an inducer and a candidate protein-protein interaction disrupting agents; assaying the cells for the non-expression and expression of the reporter coding sequence; and identifying the protein-protein interaction disrupting agent that-results in non-expression of the reporter coding sequence. The cells may be subjected to an inducer and an agent separately from selecting the cells.

The compositions and methods of the present invention may also be provided in a kit with instructions for use. In one embodiment, the kit may comprise containers or receptacle with one or more aliquots of the suppressor construct, the first interactive protein-transcriptional activation domain fusion protein coding construct, the second interactive protein- DNA binding domain fusion protein coding construct, and the reporter construct provided on one or more plasmids. The interactive proteins in the first interactive protein-transcriptional activation domain fusion protein coding construct and the second interactive protein-DNA binding domain fusion protein coding construct may be replaced, for example, by in frame multiple cloning sites for cloning any other interactive proteins whose response to a particular disrupting agent needs to be tested. The kit may also include various reagents and apparatus needed to conduct the method. In another embodiment, the kit may contain mammalian cells containing one or more components of the compositions of the present invention. In yet another embodiment, the suppressor and the reporter constructs are already introduced into the cells before introducing interactive protein constructs via either stable or transient transfection.

### EXAMPLES

### Construction of Reporter Gene Constructs

The mini promoter sequence from the immediate-early gene of human cytomegalovirus (CMV) was amplified by PCR with a sense primer containing a XbaI site and an anti-sense primer containing two restriction sites, HindIII and Hpal. The amplified sequence was inserted into pG5CAT (Clontech) at the XbaI and HpaI sites to replace the CAT gene together with the adenovirus E1b minimal promoter. The resultant plasmid was named as pG5CMV. The green fluorescent protein (GFP) gene amplified by PCR from pEGFP-C1 (Clontech) with a HindIII site at the 5' end and EcoRV and Hpa1 sites at the 3' end,was inserted into pG5CMV at the HindIII and HpaI sites, resulting in pG5CMV-GFP. A synthetic IRES sequence (Chappell et al. 2000. Proc. Nat1. Acad. Sci. USA 97, 1536-1541) with the blunt end at the 5' end and an EcoRV site at the 3' end was inserted into pG5CMV-GFP at the EcoRV site, forming pG5CMV-GFP-IRES. The β-lactamase or Lac Z gene, both of which were amplified by PCR, was inserted into pG5CMV-GFP-IRES at the EcoRV site by blunt ligation to generate either pG5CMV-GFP-IRES-β-Lac or pG5CMV-GFP-IRES-β-Gal. This construct parallels the outline shown in Figures 1D & 1E.

### Construction of Suppressor Construct

The tetracycline repressor (TetR) sequence with a HindIII site at the 5' end was amplified by PCR from PUHD15.1 (Gossen & Bujard. 1992. Proc. Natl. Acad. Sci. USA 89, 5547-5551).

The synthetic nuclear localization sequence (NLS) of 7 amino acids (Kalderon et al. 1984. Cell 39, 499- 509) with an EcoRV site at the 3' end was joined with the TetR sequence in frame by PCR. The TetR-NLS fragment was cloned into pCDNA3.1 (Invitrogen) at the HindIII and EcoRV sites. The resultant vector was termed as pCMV-TetR-NLS. The KRAB domain of human suppressor Koxl gene (Deuschle et al. 1995. Mol. Cell. Biol. 15, 1907-1914) was amplified by RT-PCR from rat kidney and cloned into pCMV-TetR-NLS in frame with NLS at the EcoRV and XbaI sites by blunt ligation to form pCMV-TetR-KRAB. This construct parallels the outline shown in Figure 1A.

### Construction of a Combined Reporter and Suppressor Construct

The suppressor construct pCMV-TetR-KRAB, as described above, was digested with BgIII and ApaI. The larger BgIII-ApaI fragment (1.8 kb) was isolated and inserted into vector pEGFP-C1 (CLONTECH) at the BgIII and Apal.sites. The resultant plasmid was termed as pEGFP-CMV-TetR-KRAB. Then, the entire reporter cassette pG5CMV-GFP-IRES-β-lac or pG5CMV-GFP-IRES-β-gal plasmids was isolated following SmaI digestion and inserted into pEGFP-CMV-TetR-KRAB at PciI and BspEI by blunt ligation. The resultant plasmid was designated as pGSCMV-GFP-IRES-β-gal-pCMV-TetR KRAB-IRES or pG5CMV-GFP-IRES-β-lac-pCMV-TetR-KRAB-IRES and used for stable transfection into cells.

### Construction of a first interactive protein-binding domain fusion gene construct and a second interactive protein-activation domain fusion gene construct

The tetracycline repressor operator sequences (4x TOP) with a SalI site at the 5' end and a XbaI site at the 3' end were synthesized and inserted at the SalI and XbaI sites of pG5CMV/Hygro that was made by inserting the hygromycin expression cassette isolated from pcDNA3.1/Hygro (Invitrogen) into the NdeI site of pG5CMV. The resultant plasmid was termed pG5CMV-tetOP. The CMV core promoter sequence containing 555 bp derived from pcDNA3.1 vector (nucleotide 232-787) was cloned into pGSCMV-tetOP at the XhoI and SalI sites between which the Gal4 binding elements (5x) were removed. The resultant plasmid was designated as pCMV-tetOP. The Gal 4 DNA binding domain (BD) and the VP16 transcriptional activation domain (AD), both of which contain the multiple cloning sites (MCS), were respectively isolated from vectors pM and pVP16 (Clontech), and cloned into pCMV-tetOP at the HindIII site in such that the other MCS were retained, but the HindIII sites was destroyed through blunt ligation. The resultant plasmids were designated as pCMV-tetOP-BD and pCMV-tetOP-AD, respectively. The interactive protein coding sequences, X and Y, were individually cloned into the appropriate sites of MCS located in the AD and BD, respectively.

### Construction of a combined first interactive protein-transcriptional activation domain fusion protein coding construct and second interactive protein-DNA binding domain fusion protein construct

The synthetic tetracycline repressor operator sequence (4xTOP) was inserted into pUHD 10.3 (Gossen & Bujard. 1992. Proc. Natl. Acad. Sci. USA 89, 5547-5551) at the XhoI and the StuI sites to replace the 7x TOP in the vector pUHD 10.3. The CMV core promoter sequence containing 550 bp, derived from pcDNA3.1 vector (nucleotide 230-780) was cloned into the XhoI site in the front of the 4xTOP, producing pUHD-CMV-tetOP. The isolated "X" protein sequence for the specific protein-protein interaction was inserted into pVP16 (Clontech) at the multiple cloning site (MCS) region to fuse with the Vp16 transcriptional activation domain (AD) in frame. The fused AD-X fragment was then isolated by the NheI and XbaI digestions, filled-in and inserted into pUHD-CMV-tetOP at the SacII and NaeI sites by blunt ligation to generate pUHD-CMV-tetOP-AD X. The synthetic IRES sequence was inserted into the pcDNA3/Hygro at the AfI II and NotI sites by blunt ligation. The resultant plasmid pcDNA3-IRES was digested with the NruI and NheI enzymes, and the CMV tetOP-AD-X fragment, isolated from pUHD-CMV-tetOP-AD-X with the NruI and XbaI digestions, was inserted into the digested vector of pcDNA3-IKES to form pCMV-tetOP-AD-X-IRES. The PCR-amplified Gal4 DNA binding domain (BD) from pM (clontech) was inserted into pcDNA3 at the NheI and XbaI sites and the isolated "Y" protein sequence for the specific protein-protein interaction was fused with the BD in frame. The BD-Y fragment was then isolated by the NheI and XbaI digestions and inserted into pCMV-tetOP-AD-X-IRES at the XbaI site to construct pCMV-tetOP-AD-X-IRES-BD-Y/Hygro. This construct parallels the outline shown in Figures 1B & 1C.

### Generation of Cell Lines Expressing Various Constructs

To establish a stable cell line expressing the individual or the combined suppressor and reporter construct, approximate 10⁵-10⁶ cells of cell line 293A were plated in a 60 mm diameter dish one day prior to transfection. The transfection with the constructs was carried out at 80% cell confluence with 5 µg of the linearized plasmid mixed with 20 µl of Superfect (Qiagen). After 48 hrs of transfection, cells were trypsinized, and diluted at 1:20 in the culture medium containing 800 µg/ml of G418 for the selection of transfected cells. After 3 weeks following the transfection, colonies were selected. The cells from the selected individual colonies were induced with Dox, and tested by co-transfection with pCMV-tet OP-AD-X-IRES-BD-Y for the protein-protein interaction that activates the reporter genes. GFP was measured 48 hrs post transfection, while the activity of β-lactamase or β-galactosidase was determined within 24-36 hrs following transfection. The cells from the selected colonies stably transfected with pG5CMV-GFP-IRBS-β-gal-pCMV-TetR-KRAB-IRES or pG5CMV-GFP-IRES-β-lac-pCMV-TetR-KRAB-IRES were further co-transfected with pCMV-tet OP-AD-X-IRES-BD-Y.

Cell lines stably expressing the individual or combined suppressor, reporter, and the interactive fusion protein constructs were further selected by G418 (400 µg/ml) and hygromycin (200 µg /ml) for screening protein-protein interaction disrupting agents. Similar procedures were applied to other mammalian cell lines such as BHK, Cos, CHO, NIH3T3 cells for establishing stable cell lines for screening protein-protein interaction disrupting agents or protein-protein interaction non-disrupting agents.

To use transient expression of interactive proteins for high throughput screening, multi-million cells stably expressing the repressor and the reporters were transfected with pCMV-tet OP-AD-X-IRES-BD-Y for overnight and then divided into 96 or 384 well plates containing both Dox and drugs. After additional 24 hrs, the expression level of the reporters was measured for identifying protein-protein interaction disrupting agents or non-disrupting agents. This method can be used for screening drugs.

### Detection of Reporter Gene Expression

***Green Fluorescence Protein (GFP).*** To directly assay the expression of GFP, cells were cultured in 96 or 384 well low fluorescence assay plates with 100 or 50 µl of DMEM medium. Dox and agents were added at concentrations described below. Following 48-hrs of treatment, the medium in each well was replaced with PBS. The intensity of GFP was directly measured at 485 nm excitation and 525 nm emission with a fluorescence multi-well plate reader (CytoFluor II, PerSeptive Biosystems or Wallac Victor V Stacker, Perkin Elmer).

***β-lactamase.*** For detecting secreted β-lactamase activity, cells in 96 or 384-well plates were grown in 100 or 50 µl of DMEM medium without phenol red. The cells were induced with Dox at a final concentration of 1 µg/ml for 12-24 hrs for reporter protein expression, 50 µl or 100 µl of reaction solution containing 100 mM phosphate, pH 7.0, 10 µM nitrocefin 0.5 µM oleic acid was added to each well. The reaction was carried out at 37°C for 30-60 minutes. In some experiments, clavulanic acid, an inhibitor for β-lactamase was added at a concentration of 25µM. β-lactamase activity was measured as an increase in absorbency at 488 nm.

***β-galactosidase.*** As with β-Lactamase activity assay, cells were grown in 100 or 50 µl DMEM medium without phenol red in 96 or 384-well plates and the substrate. After lysing the cells with lysis buffer, chlorophenol red β-D-galactophranoside (CPRG) for β-Galactosidase was added into the cell lysates at a final concentration of 100 µg /ml with Dox and agents for 24-36 hrs. The activity of the enzyme was measured as an increase of absorbency at 570 nm.

### Disruption of TGFR (RI) and FKBP12 interaction by FK506

One of the well-characterized protein-protein interactions discovered by the yeast forward two-hybrid system is the interaction of the transforming growth factor type I receptor (TGFR) (RI) with the immunophilin FKPB 12 (Wang et al. 1994. Science 265: 674-676). The FKBP 12 is a target of the macrolide FK506. It was found that FK506 competed with the TGFR (RI) for binding to FKBP12, and hence interfered with the interaction between the TGFR (RI) and FKPB12 (Wang et al. 1994. Science 265: 674-676). The mammalian reverse two-hybrid system of the present invention was tested with this protein-protein interaction pair to evaluate if the effect of FK506 on disrupting the interaction can be detected. To achieve this, the cytoplasmic domain of the TGFR (RI) was fused with the transcriptional activation domain (AD) ofVP16 and FKBP12 was fused With the Gal4 DNA binding domain (BD).

A stable cell line harboring the construct TetR-KRAB, GFP reporter construct, and the construct pCMV-tetOP-AD-RI-IRES-BD-FKBP12 was selected to evaluate the effect of drug molecule FK506 on the interaction between TGFR (RI) and FKPB12. The intensity of GFP expression was monitored following 48 hrs of treatment of the cells with the inducer Dox and the agent FK506.

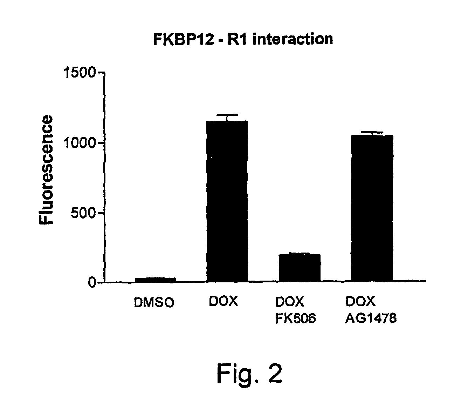

**Figure 2** shows that in the absence of Dox, expression of GFP in control (DMSO treated) cells was nearly undetectable. Addition of Dox induced expression of the interactive proteins and activated the expression of the GFP reporter gene. However, when the drug FK506 was added together with Dox, expression of GFP was almost abolished. The kinase inhibitor AG1478 (Levitzki & Gazit. 1995. Science 267: 1782-1788), an unrelated compound that does not affect the interaction of TGFR (RI) and FKPB12 exerted no effect on the Dox induced GFP expression. In obtaining results shown in Fig. 2, cells stably expressing the suppressor TetR-KRAB construct, the GFP reporter construct, and the construct pCMV-tetOP-AD-RI-IRES-BD-FKBP12 were treated with 1 µg/ml of Dox, 100 nM of FK506, and 2.5 µM of AD1478 for 48 hrs. Expression of the GFP reporter gene was evaluated following the treatment. The data represent the average and standard deviation from three independent experiments.

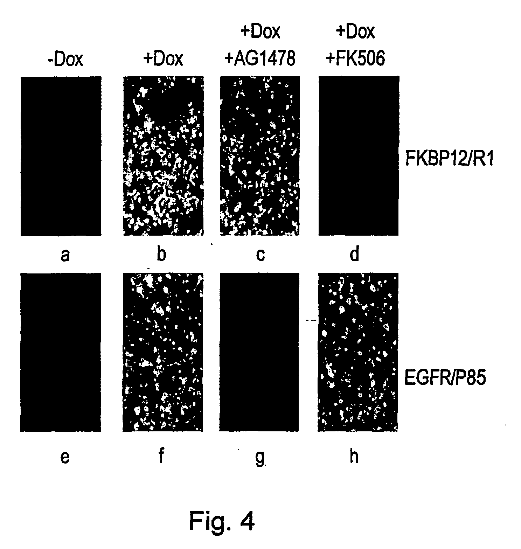

**Figures 4a to 4d** show visual evaluation of GFP expression when the construct pCMV-tetOP-AD-RI-IRES-BD-FKBP12 was transiently expressed in a stable cell line harboring the suppressor construct TetR-KRAB and the GFP reporter construct. In obtaining the results shown in Figs. 4a to 4d, cells that were stably transfected with the TetR-KRAB construct and the GFP reporter construct were transiently transfected with construct pCMV-tetOP-AD-RI-IRES-BD-FKBP12. After 12 hrs following the transfection, the inducer Dox and the drugs FK506 and AG1478 were added to the culture. Photomicrographs of the cells showing expression of GFP were taken 48 hrs after the treatment of cells. The concentration of AG1478 used was 2.5 µM and that of FK506 was 100 nM, Similar results were observed in cells stably selected for the suppressor TetR-KRAB construct, the GFP reporter construct, and the construct of pCMV-tetOP-AD-RI-IRES-BD-FKBP12.

These data demonstrated that the drug molecule FK506 effectively disrupted the interaction of FKBP12 with the receptor RI and such specific disruption of the interacting proteins by the drug was clearly detected by monitoring the expression of GFP in live cells. The stable cell line expressing the RI and FKBP12 pair of interacting proteins could be used to screen other candidate immunosupressant drugs.

### Disruption of EGFR and p85 interaction by Kinase Inhibitor AG1478

The utility of the present invention for screening potential therapeutic molecules was further demonstrated with another pair of interactive proteins, the epidermal growth factor receptor (EGFR) and the p85 subunit of phosphatidylinositol 3-kinase. It is known that the cytoplasmic domain of the EGFR family can be autophosphorylated on tyrosine residues when it is overexpressed in the nucleus (Xie & Hung. 1994. Biochem. Biophys. Res. Commun. 203: 1589-1599). The autophosphorylated EGFR recruits signaling proteins through the interaction between the phosphorylated tyrosine residues and the Src homologue 2 (SH2) domains of the signaling proteins. The subunit p85 is one of the signaling molecules that bind to the EGFR (Hu et al. 1992. Mol. Cell. Biol. 12,981-900). AG1478 is a potent inhibitor for the kinase of the EGFR (Levitzki & Gazit. 1995. Science 267: 1782-1788). We reasoned that the inhibition of the EGFR kinase activity with AG1478 would abolish the tyrosine-autophosphorylation of the receptor EGFR, resulting in the elimination of interaction between p85 and EGFR. Hence, we examined if the inhibitory activity of AG1478 could be detected by the present two-hybrid reverse system via disrupting the interaction of the EGFR and p85. As describe above, the EGFR was fused in frame with the Gal4 DNA biding domain and p85 was fused in frame with the activation domain of VP16 in frame, respectively. A stable cell line harboring the suppressor construct TetR-KRAB, the GFP reporter construct, and construct pCMV-tetOP-AD-p85-IRES-BD-EGFR was selected with appropriate antibiotics. The intensity of GFP expression was monitored following 48-hrs treatment of the cells with the inducer Dox and the drugs FK506 or AG1478.

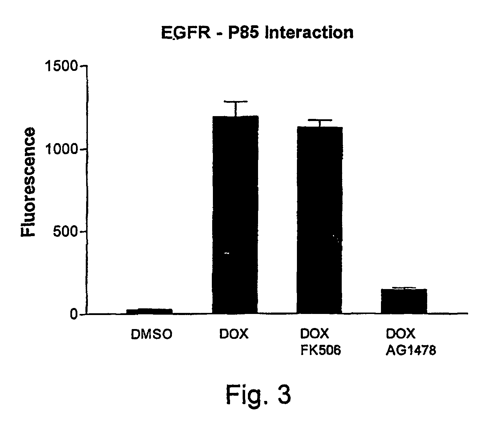

**Figure 3** shows that the expression of GFP was insignificant in control (DMSO treated) cells. Dox induced a high level of GFP expression which was not affected by FK506, an unrelated drug that does not interfere with EGFR and p85 interaction. However, addition of the EGFR kinase inhibitor AG1478 to the culture nearly completely antagonized the Dox-induced activation of the GFP reporter gene, indicating that the inhibitor disrupted the interaction between the EGFR and p85. In obtaining results shown in Fig. 3, cells stably harbouring the TetR-KRAB, GFP, and pCMV-tetOP-AD-p85-IRES-BD-EGFR constructs were treated with 1 µg/ml of Dox, 100 nM of FK506, and 2.5 µM of AD1478 for 48 hrs. Expression of the GFP was evaluated following the treatment. The data represent the average and standard deviation from three independent experiments.

**Figures 4e to 4h** show visual evaluation of GFP expression. This was done by transiently transfecting the stable cell line harbouring TetR-KRAB and GFP constructs with the pCMV-tetOP-AD-p85-IRES-BD-EGFR construct. Similar results were obtained with cells stably transfected with the suppressor construct TetR-KRAB, the GFP reporter construct and pCMV-tetOP-AD-p85-IRES-BD-EGFR. These results suggest that the reverse two-hybrid system of the present invention can be used not only to screen molecules that directly interfere with specific protein interactions but also to screen molecules that are enzyme inhibitors as long as the interaction of the enzyme with its partner protein is dependent on its enzymatic activity that can be inhibited by the inhibitor. In obtaining the results shown in Fig. 4e to 4h, cells that were stably transfected with TetR-KRAB and GFP constructs were further transiently transfected with construct pCMV-tetOP-AD-p85-IRES-BD-EGFR. After 12 hrs following the transfection, the inducer Dox and the drugs FK506 or AG 1478 were added to the culture. Photomicrographs of the cells showing expression of GFP were taken 48 hrs after the treatment of cells. The concentration of AG1478 used was 2.5 µM and that of FK506 was 100 nM.

The example described above demonstrates that the present mammalian reverse two-hybrid system also provides a convenient and powerful tool for cell-based screening of candidate inhibitors for a variety of enzymes such as proteases, phosphatases, and kinases. In particular, the system provides a convenient and powerful tool for cell-based screening of candidate inhibitors for certain disease-related kinases such as EGFR, PDGFR, Her2, VEGFR, JAKs, Met, FGFR, IGFR, insulin receptor, JAKs, ZAP70, v-Abl, Lck, Lyn and other Src -like kinases. The power and selectivity of the present system were also demonstrated in the primary screening of inhibitors for the EGFR and Her2 receptor kinases.

### Use of β-lactamase as a Reporter in the Present Invention

While GFP is a convenient reporter for direct observation of drug effects on protein-protein interactions for multiple well plate applications, it is not a sensitive reporter as the lack of enzymatic amplification requires its high level of expression for detection (10⁵ to 10⁶ molecules per cell) (Zlokarnik et al. 1998. Science 279: 84-88). For the same reason, the measurement of GFP usually cannot be performed until 48 hrs following the Dox induction. The use of an alternative reporter gene, β-lactamase, for the present reverse two-hybrid system invention is disclosed. Presently, both colorimetric and nontoxic fluorescence substrates are available for use in β-lactamase assays. Since the sensitivity of the β-lactamase reporter is determined mainly by the incubation time with its substrate, the β-lactamase-based assay is very sensitive. With a fluorescent substrate, the expression of β-lactamase at levels as low as 100 molecules per cell can be detected (Zlokarnik: et al. 1998. Science 279: 84-88). The activity of β-lactamase can be precisely determined by adding its substrate directly to the culture and measuring the consequence of the reaction such as a change of color or a large shift of fluorescence emission wavelength.

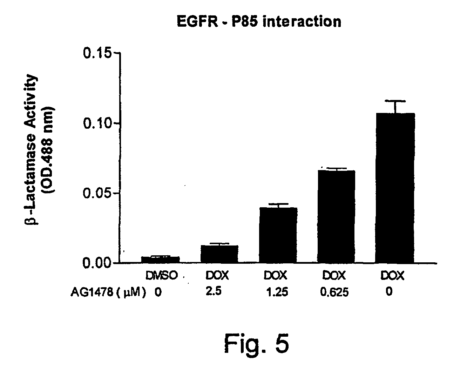

**Figure 5** shows β-lactamase activity in the medium following 12 hrs of Dox induction. The activity of β-lactamase in a 30-minute incubation displayed a dose-dependent effect of AG1478 on disrupting the interaction between EGFR and p85. In obtaining the results shown in Fig. 5, cells that were stably expressing the TetR-KRAB construct, the GFP-β-lactamase construct, and the AD-p85-IRES-BD-EGFR construct were grown in 100 or 50 µl of DMEM without Phenol Red and treated with Dox and the inhibitor AG1478 at the indicated concentrations for 12 hr. The substrate nitrocefin, in 100 or 50 µl of the reaction buffer, was added to each assay. The reaction was carried out for 30 min at 22°C. The activity of the secreted β-lactamase in the medium was directly measured by absorbency at 488 nm in a multi-well plate reader.

Similar results were obtained by using β-galactosidae as a reporter (data not shown). For the high throughput screening of drugs using the present invention, two reporter protein coding sequences comprising either GFP and β-lactamase genes or GFP and Lac Z genes were also constructed into the same plasmid by using an IRES sequence for alternative detection of the reporters.

Similar results can be obtained using a combination of the constructs pCMV-tetOP-AD-RI and pCMV-TetOP-BD-FKBP12 instead ofpCMV-tetOP-AD-IRES-BD-FKBP12.

## Claims

1. A mammalian reverse two-hybrid system for identifying a change in protein-protein interaction comprising:
a suppressor construct comprising a constitutive promoter operably linked to a repressor protein coding sequence;
an interactive fusion protein coding construct comprising a promoter operably linked to a repressible operator repressible by a repressor protein coded by said repressor protein coding sequence, a first interactive protein coding sequence fused in frame with a transcriptional activation domain protein coding sequence and a second interactive protein coding sequence fused in frame with a DNA binding domain protein coding sequence; and,
a reporter construct comprising a DNA binding element bindable to said DNA binding domain protein operably linked to a minimal promoter and a reporter coding sequence.

2. The mammalian reverse two-hybrid system according to claim 1 wherein said interactive fusion protein coding construct comprises:
a first interactive protein-transcriptional activation domain fusion protein coding construct comprising a promoter operably linked to a repressible operator repressible by a repressor protein coded by said repressor protein coding sequence and a first interactive protein coding sequence fused in frame with a transcriptional activation domain protein coding sequence; and,
a second interactive protein-DNA binding domain fusion protein gene construct comprising a promoter operably linked to a repressible operator repressible by a repressor protein coded by said repressor protein coding sequence, a second interactive protein coding sequence fused in frame with a DNA binding domain protein coding sequence.

3. The mammalian reverse two-hybrid system according to claim 1 additionally comprising at least one internal ribosome entry site (IRES) sequence.

4. The mammalian reverse two-hybrid system according to claim 1, wherein said first interactive protein coding sequence fused in frame with a transcriptional activation domain protein coding sequence is operably linked to said second interactive protein coding sequence fused in frame with a DNA binding domain protein coding sequence through an internal ribosome entry site (IRES) sequence.

5. The mammalian reverse two-hybrid system according to claim 1, wherein said reporter construct additionally comprises a second reporter coding sequence operably linked to a said reporter coding sequence.

6. The mammalian reverse two-hybrid system according to claim 5, wherein said first reporter coding sequence is operably linked to said second reporter coding sequence through an internal ribosome entry site (IRES) sequence.

7. The mammalian reverse two-hybrid system according to claim 1 wherein in said suppressor construct said constitutive promoter is selected from the group consisting of promoters from human cytomegalovirus (CMV), SV40, respiratory syncytial virus (RSV), EF1, and thymine kinase (TK) genes.

8. The mammalian reverse two-hybrid system according to claim 1 wherein in said suppressor construct said repressor protein coding sequence codes for a protein selected from the group consisting of Lac repressor, λ repressor, Tet repressor, and hybrid-Tet repressor (TetR).

9. The mammalian reverse two-hybrid system according to claim 8, wherein said hybrid-TetR has the TetR protein linked with the KRAB domain of the human transcription suppressor Kox1 by a nuclear localization sequence (NLS).

10. The mammalian reverse two-hybrid system according to claim 1 wherein said suppressor construct additionally comprises a drug selective marker which provides resistance to a drug.

11. The mammalian reverse two-hybrid system according to claim 1 wherein in said interactive fusion protein coding construct said promoter and repressible operator are selected from the group consisting of a core human cytomegalovirus (CMV), SV40, respiratory syncytial virus (RSV), EF1 and thymine kinase (TK) promoter linked with tetracycline operator.

12. The mammalian reverse two-hybrid system according to claim 1 wherein said interactive fusion protein coding construct codes for a protein selected from the group consisting of receptors, adapters, affectors and enzymes.

13. The mammalian reverse two-hybrid system according to claim 12, wherein said first interactive protein coding sequence codes for a disease-related signalling molecule.

14. The mammalian reverse two-hybrid system according to claim 1 wherein said transcriptional activation domain coding sequence is selected from the group consisting of GAL4, B42, VP16 and NF-kB p65 coding sequences.

15. The mammalian reverse two-hybrid system according to claim 1 wherein said second interactive protein coding sequence codes for a protein selected from the group consisting of a protease, a phosphatase, a kinase, a receptor and an enzyme.

16. The mammalian reverse two-hybrid system according to claim 15, wherein said kinase is a disease-related kinase.

17. The mammalian reverse two-hybrid system according to claim 16, wherein said disease-related kinase is selected from the group consisting of EGFR, PDGFR, Her2, VEGFR, Met, FGFR, IGFR, insulin receptor, JAKs, PKA, PKC, MAP kinase, ZAP70, v-Abl, and Lck, Lyn, or other Src-like kinase.

18. The mammalian reverse two-hybrid system according to claim 1 wherein the DNA binding domain coding sequence is selected from the group consisting of Gal4 DNA binding domain, Lac repressor, Tet repressor, lambda repressor, Lax A DNA binding sequence, NF-kB p65 and p53.

19. The mammalian reverse two-hybrid system according to claim 3 or 5, wherein in said internal ribosome entry site (IRES) sequence is selected from the group consisting of a synthetic IRES sequence and an encephalomyocarditis virus (EMCV) IRES.

20. The mammalian reverse two-hybrid system according to claim 1 wherein in said reporter construct said DNA binding element is selected from the group consisting of Gal4 binding element, Lac operator, Tet operator, lambda operator, Lax A binding element and p53 binding sites.

21. The mammalian reverse two-hybrid system according to any one of claims 1,4, and 5, wherein in said reporter construct said promoter is selected from the group consisting of a minimal promoter from human cytomegalovirus (CMV) promoter, SV40 early promoter, adenovirus Elb promoter, respiratory syncytial virus (RSV) promoter, EF1 promoter and thymine kinase (TK) promoter.

22. The mammalian reverse two-hybrid system according to any one of claims 1, 4, and 5, wherein said reporter coding sequence codes for a protein selected from the group consisting of a green fluorescent protein (GFP) or GFP-active derivative, a luciferase, a β-lactamase, a β-galactosidase, an alkaline phosphatase and chloramphenicol acetyl transferase.

23. A plasmid comprising the mammalian reverse two-hybrid system according to claim 1.

24. A two-plasmid set comprising the mammalian reverse two-hybrid system according to claim 1, wherein said suppressor construct and said reporter construct are provided on a first plasmid and said first interactive protein-transcriptional activation domain fusion protein coding construct and said second interactive protein-DNA binding domain fusion protein coding construct together are provided on a second plasmid.

25. A mammalian cell comprising a mammalian reverse two-hybrid system according to claim 1.

26. A mammalian cell according to claim 25 wherein said mammalian reverse two-hybrid system is integrated into a chromosome in said cell.

27. A method for identifying a change in protein-protein interaction comprising:
subjecting a mammalian reverse two-hybrid system according to any one of claims 1 to 22 in a cell to an inducer and a test protein-protein interaction disrupting agent;
and assaying said cell for expression or non-expression of said reporter coding sequence, non-expression of said reporter coding sequence being indicative of disrupting ability of said test protein-protein interaction disrupting agent

28. The method of claim 27 wherein said disrupting agent is an indirect disrupting agent being tested for an ability to inhibit activity of a disease-related enzyme.

29. A method for identifying a change in protein-protein interaction comprising:
(a) stably introducing a suppressor construct and a reporter construct in a cell;
(b)(i) stably introducing a first interactive protein-transcriptional activation domain fusion protein coding construct and a second interactive protein-DNA binding domain fusion protein coding construct into said cell; or
(b)(ii) transiently introducing a first interactive protein-transcriptional activation domain fusion protein coding construct and a second interactive protein-DNA binding domain fusion protein coding construct into said cell;
(c) subjecting a cell resulting from step (b)(i) or (b)(ii) to an inducer and a test protein-protein interaction disrupting agent;
(d) assaying said subjected cell for the non-expression and expression of said reporter coding sequence; and,
(e) identifying said protein-protein interaction disrupting agent based on non-expression of said reporter coding sequence.

30. The method of identifying a change in protein-protein interaction according to claim 28 wherein said cell is a mammalian cell.

31. A kit for identifying a change in protein-protein interaction comprising:
one or more aliquots of a suppressor construct, a first interactive protein-transcriptional activation domain fusion protein coding construct, a second interactive protein-DNA binding domain fusion protein coding construct, and a reporter construct on one or more plasmids; and,
one or more aliquots of a reagent, cell, and apparatus needed for conducting a method for identifying a change in protein-protein interaction.

32. A kit for identifying a change in protein-protein interaction comprising:
one or more aliquots of one or more constructs on one or more plasmids as defined in claim 31;
one or more aliquots of a cell containing one or more constructs as defined in claim 31; and,
one or more aliquots of a reagent and apparatus needed for conducting a method for identifying a change in protein-protein interaction.

## Patentansprüche

1. Reverses Säugetier-Zweihybridsystem zur Identifizierung einer Veränderung in Protein-Protein-Wechselwirkungen mit:
einem Suppressorkonstrukt, einen operativ an eine Repressorprotein-Kodiersequenz gebundenen konstitutiven Promotor umfassend;
einem Kodierkonstrukt für ein interaktives Fusionsprotein, umfassend einen operativ an einen repressiblen Operator gebundenen Promotor, der durch ein Repressorprotein unterdrückt werden kann, das durch die Repressorprotein-Kodiersequenz kodiert wird, eine erste Kodiersequenz für ein interaktives Protein, die in Frame mit einer Proteinkodiersequenz für eine transkriptionelle Aktivierungsdomäne fusioniert ist, und eine zweite Kodiersequenz für ein interaktives Protein, die in Frame mit einer Proteinkodiersequenz für eine DNA-Bindungsdomäne fusioniert ist; und
einem Reporterkonstrukt mit einem operativ an einen Minimalpromotor gebundenen, DNA bindenden Element, welches das DNA-Bindungsdomänenprotein binden kann, sowie einer Reporterkodiersequenz.

2. Reverses Säugetier-Zweihybridsystem nach Anspruch 1, **dadurch gekennzeichnet, dass** das Kodierkonstrukt für das interaktive Fusionsprotein umfasst:
ein erstes Kodierkonstrukt für ein Fusionsprotein aus einem interaktiven Protein und einer transkriptionellen Aktivierungsdomäne mit einem operativ an einen repressiblen Operator gebundenen Promotor, der durch ein Repressorprotein unterdrückt werden kann, das durch die Repressorprotein-Kodiersequenz kodiert wird, und einer ersten Kodiersequenz für ein interaktives Protein, die in Frame mit einer Proteinkodiersequenz für eine transkriptionelle Aktivierungsdomäne fusioniert ist; und
ein zweites Genkonstrukt für ein Fusionsprotein aus einem interaktiven Protein und einer transkriptionellen Aktivierungsdomäne mit einem operativ an einen repressiblen Operator gebundenen Promotor, der durch ein Repressorprotein unterdrückt werden kann, das durch die Repressorprotein-Kodiersequenz kodiert wird, und einer zweiten Kodiersequenz für ein interaktives Protein, die in Frame mit einer Proteinkodiersequenz für eine DNA-Bindungsdomäne fusioniert ist.

3. Reverses Säugetier-Zweihybridsystem nach Anspruch 1, zusätzlich zumindest eine Sequenz interner Ribosomen-Eintrittsstellen (IRES) umfassend.

4. Reverses Säugetier-Zweihybridsystem nach Anspruch 1, **dadurch gekennzeichnet, dass** die in Frame mit einer Proteinkodiersequenz für eine transkriptionelle Aktivierungsdomäne fusionierte Kodiersequenz für interaktive Proteine durch eine Sequenz interner Ribosomen-Eintrittsstellen (IRES) operativ an die in Frame mit einer Proteinkodiersequenz für eine DNA-Bindungsdomäne fusionierte zweite Kodiersequenz für interaktive Proteine gebunden ist.

5. Reverses Säugetier-Zweihybridsystem nach Anspruch 1, **dadurch gekennzeichnet, dass** das Reporterkonstrukt zusätzlich eine zweite, operativ an die benannte Reporterkodiersequenz gebundene Reporterkodiersequenz umfasst.

6. Reverses Säugetier-Zweihybridsystem nach Anspruch 5, **dadurch gekennzeichnet, dass** die erste Reporterkodiersequenz über eine Sequenz interner Ribosomen-Eintrittsstellen (IRES) operativ an die zweite Reporterkodiersequenz gebunden ist.

7. Reverses Säugetier-Zweihybridsystem nach Anspruch 1, **dadurch gekennzeichnet, dass** der konstitutive Promotor im Suppressorkonstrukt aus der Gruppe ausgewählt ist, die aus Promotoren des humanen Cytomegalovirus (CMV), des SV40, des respiratorischen Synzytialvirus (RSV), des EF1 und Thyminkinase- (TK) Genen besteht.

8. Reverses Säugetier-Zweihybridsystem nach Anspruch 1, **dadurch gekennzeichnet, dass** die Repressorprotein-Kodiersequenz im Suppressorkonstrukt für ein Protein kodiert, das aus der Gruppe ausgewählt ist, die aus Lac-Repressor, λ-Repressor, Tet-Repressor und hybridem Tet-Repressor (TetR) besteht.

9. Reverses Säugetier-Zweihybridsystem nach Anspruch 8, **dadurch gekennzeichnet, dass** bei dem hybriden TetR das TetR-Protein durch eine Kernlokalisierungssequenz (NLS) mit der KRAB-Domäne des humanen Transkriptionssuppressors Kox 1 verbunden ist.

10. Reverses Säugetier-Zweihybridsystem nach Anspruch 1, **dadurch gekennzeichnet, dass** das Suppressorkonstrukt zusätzlich einen arzneimittel-selektiven Marker umfasst, der Resistenz gegenüber einem Arzneimittel liefert.

11. Reverses Säugetier-Zweihybridsystem nach Anspruch 1, **dadurch gekennzeichnet, dass** der Promotor und der repressible Operator im interaktiven Fusionsprotein-Kodierkonstrukt aus der Gruppe ausgewählt sind, die aus einem mit dem Tetracyclin-Operator verbundenen Kempromotor des humanen Cytomegalovirus (CMV), des SV40, des respiratorischen Synzytialvirus (RSV), des EF1 und der Thyminkinase (TK) besteht.

12. Reverses Säugetier-Zweihybridsystem nach Anspruch 1, **dadurch gekennzeichnet, dass** das Kodierkonstrukit für interaktive Fusionsproteine für ein Protein kodiert, das aus der aus Rezeptoren, Adaptoren, Effektoren und Enzymen bestehenden Gruppe ausgewählt ist.

13. Reverses Säugetier-Zweihybridsystem nach Anspruch 12, **dadurch gekennzeichnet, dass** die erste Kodiersequenz für interaktive Proteine für ein krankheitsbezogenes Signalmolekül kodiert.

14. Reverses Säugetier-Zweihybridsystem nach Anspruch 1, **dadurch gekennzeichnet, dass** die Kodiersequenz für transkriptionelle Aktivierungsdomänen aus der Gruppe ausgewählt ist, die aus den Kodiersequenzen GAL4, B42, VP16 und NF-kB p65 besteht.

15. Reverses Säugetier-Zweihybridsystem nach Anspruch 1, **dadurch gekennzeichnet, dass** die zweite Kodiersequenz für interaktive Proteine für ein Protein kodiert, das aus der Gruppe ausgewählt ist, die aus einer Protease, einer Phosphatase, einer Kinase, einem Rezeptor und einem Enzym besteht.

16. Reverses Säugetier-Zweihybridsystem nach Anspruch 15, **dadurch gekennzeichnet, dass** die Kinase eine krankheitsbezogene Kinase ist.

17. Reverses Säugetier-Zweihybridsystem nach Anspruch 16, **dadurch gekennzeichnet, dass** die krankheitsbezogene Kinase aus der Gruppe ausgewählt ist, die aus EGFR, PDGFR, Her2, VEGFR, Met, FGFR, IGFR, Insulinrezeptor, JAKs, PKA, PKC, MAP-Kinase, ZAP70, v-Abl sowie Lck, Lyn oder anderen Src-artigen Kinasen besteht.

18. Reverses Säugetier-Zweihybridsystem nach Anspruch 1, **dadurch gekennzeichnet, dass** die Kodiersequenz für die DNA-Bindungsdomäne aus der Gruppe ausgewählt ist, die aus Gal4-DNA-Bindungsdomäne, Lac-Repressor, Tet-Repressor, Lambda-Repressor, Lax A-DNA-Bindungssequenz, NF-kB p65 und p53 besteht.

19. Reverses S äugetier-Zweihybridsystem nach Anspruch 3 oder 5, **dadurch gekennzeichnet, dass** die Sequenz interner Ribosomen-Eintrittsstellen (IRES) aus der Gruppe ausgewählt ist, die aus einer synthetischen IRES-Sequenz und einer Encephalomyocarditisvirus- (EMCV-) IRES besteht.

20. Reverses Säugetier-Zweihybridsystem nach Anspruch 1, **dadurch gekennzeichnet, dass** das DNA-Bindungselement im Reporterkonstrukt. aus der Gruppe ausgewählt ist, die aus Gal4-Bindungselement, Lac-Operator, Tet-Operator, Lambda-Operator, Lax A-Bindungselement und p53-Bindungsstellen besteht.

21. Reverses Säugetier-Zweihybridsystem nach einem beliebigen der Ansprüche 1, 4 und 5, **dadurch gekennzeichnet, dass** der Promotor im Reporterkonstrukt aus der Gruppe ausgewählt ist, die aus einem Minimalpromotor aus humanem Cytomegalovirus- (CMV-) promotor, frühem SV40-Promotor, Adenovirus E1b-Promotor, respiratorischem Synzytialvirus- (RSV-) Promotor, EF1-Prornotor und Thyminkinase- (TK-) Promotor besteht.

22. Reverses Säugetier-Zweihybridsystem nach einem beliebigen der Ansprüche 1, 4 und 5, **dadurch gekennzeichnet, dass** die Reporterkodiersequenz für ein Protein kodiert, das aus der Gruppe ausgewählt ist, die aus einem grün fluoreszierenden Protein (GFP) oder einem GFP-aktiven Abkömmling, einer Luciferase, einer β-Lactamase, einer β-Galactosidase, einer alkalischen Phosphatase und Chloramphenicol-Acetyltransferase besteht.

23. Plasmid, das reverse Säugetier-Zweihybridsystem nach Anspruch 1 umfassend.

24. Satz aus zwei Plasmiden, das reverse Säugetier-Zweihybridsystem nach Anspruch 1 umfassend, **dadurch gekennzeichnet, dass** das Suppressorkonstrukt sowie das Reporterkonstrukt auf einem ersten Plasmid zur Verfügung stehen und das erste Kodierkonstrukt für Fusionsproteine aus interaktiven Proteinen und einer transkriptionellen Aktivierungsdomäne sowie das zweite Kodierkonstrukt für Fusionsproteine aus interaktiven Proteinen und einer DNA-Bindungsdomäne zusammen auf einem zweiten Plasmid zur Verfügung stehen.

25. Säugetierzelle, ein reverses Säugetier-Zweihybridsystem nach Anspruch 1 umfassend.

26. Säugetierzelle nach Anspruch 25, **dadurch gekennzeichnet, dass** das reverse Säugetier-Zweihybridsystem in ein Chromosom in dieser Zelle integriert ist.

27. Verfahren zur Identifizierung einer Veränderung in Protein-Protein-Wechselwirkungen, umfassend:
ein reverses *Säugetier-Zweihybridsystems* nach einem beliebigen der Ansprüche 1 bis 22 in einer Zelle einem Induktor und einer zu prüfenden, Protein-Protein-Wechselwirkungen störenden Substanz auszusetzen;
und die Zelle auf die Expression oder Nichtexpression der Reporterkodiersequenz zu prüfen, wobei Nichtexpression der Reporterkodiersequenz eine Störfähigkeit der zu prüfenden, Protein-Protein-Wechselwirkungen störenden Substanz anzeigt.

28. Verfahren nach Anspruch 27, **dadurch gekennzeichnet, dass** die störende Substanz eine indirekt störende Substanz ist, die auf ihre Fähigkeit geprüft wird, die Aktivität eines krankheitsbezogenen Enzyms zu hemmen.

29. Verfahren zur Identifizierung einer Veränderung in Protein-Protein-Wechselwirkungen, umfassend:
a) ein Suppressorkonstrukt und ein Reporterkonstrukt stabil: in eine Zelle einzuführen;
b.1) ein erstes Kodierkonstrukt für Fusionsproteine aus interaktiven Proteinen und einer transkriptionellen Aktivierungsdomäne sowie ein zweites Kodierkonstrukt für Fusionsproteine aus interaktiven Proteinen und einer DNA-Bindungsdomäne in die Zelle einzuführen; oder
b.2) ein erstes Kodierkonstrukt für Fusionsproteine aus interaktiven Proteinen und einer transkriptionellen Aktivierungsdomäne sowie ein zweites Kodierkonstrukt für Fusionsproteine aus interaktiven Proteinen und einer DNA-Bindungsdomäne vorübergehend in die Zelle einzuführen;
c) eine aus Schritt b,1 oder b.2 gewonnene Zelle einem Induktor und einer zu prüfenden, Protein-Protein-Wechselwirkungen störenden Substanz auszusetzen;
d) die ausgesetzte Zelle auf die Nichtexpression oder Expression der Reporterkodiersequenz zu prüfen; und
e) auf der Basis der Nichtexpression der Reporterkodiersequenz die die Protein-Protein-Wechselwirkungen störende Substanz zu identifizieren.

30. Verfahren zur Identifizierung einer Veränderung in Protein-Protein-Wechselwirkungen nach Anspruch 28, **dadurch gekennzeichnet, dass** die Zelle eine Säugetierzelle ist.

31. Reagentiensatz zur Identifizierung einer Veränderung in Protein-Protein-Wechselwirkungen mit:
einem oder mehreren aliquoten Teilen eines Suppressorkonstrukts, einem ersten Kodierkonstrukt für Fusionsproteine aus interaktiven Proteinen und einer transkriptionellen Aktivierungsdomäne, einem zweiten Kodierkonstrukt für Fusionsproteine aus interaktiven Proteinen und einer DNA-Bindungsdomäne und einem Reporterkonstrukt auf einem oder mehreren Plasmiden; und
einem oder mehreren aliquoten Teilen eines Reagens, einer Zelle sowie Gerätschaften, die benötigt werden, um ein Verfahren zur Identifizierung einer Veränderung in Protein-Protein-Wechselwirkungen auszuführen.

32. Reagentiensatz zur Identifizierung einer Veränderung in Protein-Protein-Wechselwirkungen mit:
einem oder mehreren aliquoten Teilen eines oder mehrerer Konstrukte auf einem oder mehreren Plasmiden, wie in Anspruch 31 definiert;
einem oder mehreren aliquoten Teilen einer Zelle, die ein oder mehrere Konstrukte enthält, wie in Anspruch 31 definiert; und
einem oder mehreren aliquoten Teilen eines Reagens sowie Gerätschaften, die benötigt werden, um ein Verfahren zur Identifizierung einer Veränderung in Protein-Protein-Wechselwirkungen auszuführen.

## Revendications

1. Système double hybride inverse de mammifère destiné à l'identification d'un changement d'interaction protéine-protéine comprenant :
une construction suppressive comprenant un promoteur constitutif lié de façon opérationnelle à une séquence codante d'une protéine répresseur ;
une construction codant pour une protéine de fusion interactive comprenant un promoteur lié de façon opérationnelle à opérateur répressible, répressible par une protéine répresseur codée par ladite séquence codante de la protéine répresseur, une séquence codante d'une première protéine interactive fusionnée en cadre de lecture avec une séquence codante d' un domaine protéique d'activation de la transcription, et une séquence codante d'une seconde protéine interactive fusionnée en cadre de lecture avec une séquence codante d'un domaine protéique de liaison à l'ADN ; et,
une construction de rapporteur comprenant un élément de liaison à l'ADN pouvant se lier audit domaine protéique de liaison à l'ADN, lié de façon opérationnelle à un promoteur minimal et à une séquence codante du rapporteur.

2. Système double hybride inverse de mammifère selon la revendication 1, dans lequel ladite construction codant pour la protéine de fusion interactive comprend :
une construction codant pour une protéine de fusion « première protéine interactive/domaine d'activation de la transcription » comprenant un promoteur lié de façon opérationnelle à opérateur répressible, répressible par une protéine répresseur codée par ladite séquence codante de la protéine répresseur, et une séquence codante d'une première protéine interactive fusionnée en cadre de lecture avec une séquence codante d'un domaine protéique d'activation de la transcription ; et,
une construction de gène d'une protéine de fusion « seconde protéine interactive/domaine de liaison à l'ADN » comprenant un promoteur lié de façon opérationnelle à opérateur répressible, répressible par une protéine répresseur codée par ladite séquence codante de la protéine répresseur, et une séquence codante d'une seconde protéine interactive fusionnée en cadre de lecture avec une séquence codante d'un domaine protéique de liaison à l'ADN.

3. Système double hybride inverse de mammifère selon la revendication 1, comprenant de plus au moins une séquence du site d'entrée interne du ribosome (IRES).

4. Système double hybride inverse de mammifère selon la revendication 1, dans lequel ladite séquence codante d'une première protéine interactive fusionnée en cadre de lecture avec une séquence codante d'un domaine protéique d'activation de la transcription est liée de façon opérationnelle à ladite séquence codante d'une seconde protéine interactive fusionnée en cadre de lecture avec une séquence codante d'un domaine protéique de liaison à l'ADN, par l'intermédiaire d'une séquence du site d'entrée interne du ribosome (IRES).

5. Système double hybride inverse de mammifère selon la revendication 1, dans lequel ladite construction de rapporteur comprend en plus une séquence codante d'un second rapporteur lié de façon opérationnelle à ladite séquence codante du rapporteur.

6. Système double hybride inverse de mammifère selon la revendication 5, dans lequel ladite séquence codante du premier rapporteur est liée de façon opérationnelle à ladite séquence codante du second rapporteur par l'intermédiaire d'une séquence du site d'entrée interne du ribosome (IRES).

7. Système double hybride inverse de mammifère selon la revendication 1, dans lequel on choisit ledit promoteur constitutif de ladite construction suppressive dans le groupe constitué par les promoteurs du cytomégalovirus humain (CMV), de SV40, du virus syncytial respiratoire (RSV), des gènes d'EF1 et de la thymine kinase (TK).

8. Système double hybride inverse de mammifère selon La revendication 1, dans lequel ladite séquence codante de la protéine répresseur de la construction suppressive code pour une protéine choisie dans le groupe constitué par le répresseur Lac, le répresseur λ, le répresseur Tet, et le répresseur Tet hybride (TetR).

9. Système double hybride inverse de mammifère selon la revendication 8, dans lequel ledit hybride TetR a la protéine TetR liée au domaine KRAB du suppresseur de transcription humain Kox1 par une séquence de localisation nucléaire(SLN).

10. Système double hybride inverse de mammifère selon la revendication 1, dans lequel la construction suppressive comprend en plus un marqueur sélectif à une drogue, fournissant la résistance à une drogue.

11. Système double hybride inverse de mammifère selon la revendication 1, dans lequel on choisit ledit promoteur et ledit opérateur répressible de ladite construction codant pour la protéine de fusion interactive, dans le groupe constitué par le promoteur du centre du cytomégalovirus humain (CMV), de SV40, du virus syncytial respiratoire (RSV), d'EF1 et de la thymine kinase (TK) lié à l'opérateur de la tétracycline.

12. Système double hybride inverse de mammifère selon la revendication 1, dans lequel ladite construction codant pour la protéine de fusion interactive code pour une protéine choisie dans le groupe constitué par des récepteurs, des adaptateurs, des effecteurs et des enzymes.

13. Système double hybride inverse de mammifère selon la revendication 12, dans lequel ladite séquence codante de la première protéine interactive code pour une molécule de signal liée à une maladie.

14. Système double hybride inverse de mammifère selon la revendication 1, dans lequel on choisit ladite séquence codante du domaine d'activation de la transcription dans le groupe constitué par les séquences codantes de GAL4, B42, VP16 et NF-kB de p65.

15. Système double hybride inverse de mammifère selon la revendication 1, dans lequel ladite séquence codante de la seconde protéine interactive code pour une protéine choisie dans le groupe constitué par une protéase, une phosphatase, une kinase, un récepteur et une enzyme.

16. Système double hybride inverse de mammifère selon la revendication 15, dans lequel ladite kinase est une kinase liée à une maladie.

17. Système double hybride inverse de mammifère selon la revendication 16, dans lequel on choisit ladite kinase liée à une maladie dans le groupe constitué par EGFR, PDGFR, Her2, VEGFR, Met, FGFR, IGFR, le récepteur de l'insuline, JAK, PKA, PKC, MAP kinase, ZAP70, Abl-v, et Lck, Lyn ou d'autres kinases de type Src.

18. Système double hybride inverse de mammifère selon la revendication 1, dans lequel on choisit la séquence codante du domaine de liaison à l'ADN dans le groupe constitué par le domaine de liaison à l'ADN de Ga14, le répresseur Lac, le répresseur Tet, le répresseur lambda, le domaine de liaison à l'ADN de LaxA, NF-kB de p65 et de p53.

19. Système double hybride inverse de mammifère selon la revendication 3 ou 5, dans lequel on choisit ladite séquence du site d'entrée interne du ribosome (IRES) dans le groupe constitué par une séquence IRES synthétique et un IRES du virus de l'encéphalomyocardite (EMCV).

20. Système double hybride inverse de mammifère selon la revendication 1, dans lequel on choisit ledit élément de liaison à l'ADN de ladite construction de rapporteur dans le groupe constitué par l'élément de liaison Gal4, l'opérateur Lac, l'opérateur Tet, l'opérateur lambda, de l'élément de liaison LaxA, et des sites de liaison de p53.

21. Système double hybride inverse de mammifère selon l'une quelconque des revendications 1, 4 et 5, dans lequel on choisit ledit promoteur de ladite construction de rapporteur dans le groupe constitué par un promoteur minimal du cytomégalovirus humain (CMV), un promoteur précoce de SV40, un promoteur E1b d'un adénovirus, un promoteur du virus syncytial respiratoire (RSV), le promoteur EF1 et le promoteur de la thymine kinase (TK).

22. Système double hybride inverse de mammifère selon l'une quelconque des revendications 1, 4 et 5, dans lequel ladite séquence du rapporteur code pour une protéine choisie dans le groupe constitué par une protéine fluorescente verte (GFP) ou d'un dérivé actif de GFP, une luciférase, une β lactamase, une β-galactosidase, une phosphatase alcaline et la chloramphénicol acétyl-transférase.

23. Plasmide comprenant le système double hybride inverse de mammifère selon la revendication 1.

24. Jeu de deux plasmides comprenant le système double hybride inverse de mammifère selon la revendication 1, dans lequel on fournit ladite construction suppressive et ladite construction de rapporteur sur un premier plasmide, et on fournit ensemble ladite construction codant pour une protéine de fusion « première protéine interactive/domaine d'activation de la transcription » et ladite construction codant pour une protéine de fusion « seconde protéine interactive/domaine de liaison à l'ADN » sur un second plasmide.

25. Cellule de mammifère comprenant le système double hybride inverse de mammifère selon la revendication 1.

26. Cellule de mammifère selon la revendication 25, dans laquelle on intègre ledit système double hybride inverse de mammifère dans un chromosome de ladite cellule.

27. Procédé destiné à identifier un changement dans une interaction protéine-protéine comprenant :
là soumission d'un système double hybride inverse de mammifère selon une quelconque des revendications 1 à 22 dans une cellule, à un inducteur et à un test par agent perturbateur d'une interaction protéine-protéine ;
et le dosage de ladite cellule pour l'expression ou la non-expression de ladite séquence codante du rapporteur, la non-expression de ladite séquence codante du rapporteur étant indicative de la capacité de rupture dudit agent perturbateur d'une interaction protéine-protéine du test.

28. Procédé selon la revendication 27, dans lequel ledit agent perturbateur est un agent perturbateur indirect qui est testé pour une capacité à inhiber l'activité d'une enzyme liée à une maladie.

29. Procédé destiné à identifier un changement dans une interaction protéine-protéine comprenant :
(a) l'introduction de façon stable d'une construction suppressive et d'une construction de rapporteur dans une cellule ;
(b)(i) l'introduction de façon stable d'une construction codant pour une protéine de fusion « première protéine interactive/domaine d'activation de la transcription » et d'une construction codant pour une protéine de fusion « seconde protéine interactive/domaine de liaison à l'ADN » dans ladite cellule ; ou
(b)(ii) l'introduction de façon transitoire d'une construction codant pour une protéine de fusion « première protéine interactive/domaine d'activation de la transcription » et d'une construction codant pour une protéine de fusion « seconde protéine interactive/domaine de liaison à l'ADN » dans ladite cellule ;
(c) la soumission d'une cellule résultante de l'étape (b)(i) ou (b)(ii) à un inducteur et à un test par agent perturbateur d'une interaction protéine-protéine ;
(d) le dosage de ladite cellule soumise pour la non-expression et l'expression de ladite séquence codante du rapporteur ;et ;
(e) l'identification dudit agent perturbateur d'une interaction protéine-protéine sur la base de la non-expression de ladite séquence codante du rapporteur.

30. Procédé destiné à identifier un changement dans une interaction protéine-protéine selon la revendication 28, dans lequel ladite cellule est une cellule de mammifère.

31. Kit destiné à identifier un changement dans une interaction protéine-protéine comprenant :
Un ou plusieurs aliquotes d'une construction suppressive, d'une construction codant pour une protéine de fusion « première protéine interactive/domaine d'activation de la transcription » et d'une construction codant pour une protéine de fusion « seconde protéine interactive/ domaine de liaison à l'ADN », et d'une construction de rapporteur sur un ou plusieurs plasmides ;et,
Un ou plusieurs aliquotes d'un réactif, d'une cellule ou d'appareil dont on a besoin pour mettre en oeuvre un procédé destiné à identifier un changement dans une interaction protéine-protéine.

32. Kit destiné à identifier un changement dans une interaction protéine-protéine comprenant:
Un ou plusieurs aliquotes d'une ou plusieurs constructions sur un ou plusieurs plasmides comme défini selon la revendication 31 ;
Un ou plusieurs aliquotes d'une cellule contenant une ou plusieurs constructions comme défini selon la revendication 31 ; et
Un ou plusieurs aliquotes d'un réactif ou d'un appareil dont on a besoin pour mettre en oeuvre un procédé destiné à identifier un changement dans une interaction protéine-protéine.
